# EUROPEAN PATENT APPLICATION

(11) **EP 4 245 363 A1**
(43) Date of publication of application: **20.09.2023**
(21) Application number: 22163081.7
(22) Date of filing: 18.03.2022
(51) Int. Cl.: A61N 5/10

(54) **A PARTICLE BEAM TRANSPORT SYSTEM FOR THE DELIVERY OF PARTICLE BEAM THERAPY**

(71) Applicant: PAUL SCHERRER INSTITUT, 5232 Villigen PSI (CH)
(72) Inventor: MARADIA, Vivek, 8142 Uitikon Waldegg (CH); MEER, David, 5200 Brugg (CH); PSOROULAS, Serena, 8050 Zürich (CH); BAUMGARTEN, Christian, 5244 Birrhard (CH); LOMAX, Antony, 5210 Windisch (CH)
(74) Representative: Fischer, Michael

(57) **Abstract**

The present invention is related to a particle therapy apparatus used for particle therapy. More particularly, this invention relates to a beamline and gantry comprising an energy/momentum selection system. The present invention shows a new design of an energy selection system (ESS) for cyclotron-based particle therapy beamline. This invention provides a way to reduce the momentum spread of the beam using an energy attenuation member, preferably in form of a wedge, without introducing significant beam losses. As a result, it allows for the transport of a higher number of particles through the beamline and maximizes the transmission between cyclotron and patient location for particle therapy.

## Description

The present invention relates to a particle beam transport system for the delivery of particle beam therapy to a patient. More particularly, this invention relates to a energy/momentum selection system to reduce the energy/momentum spread of the beam without cutting parts of the beam.

Over the past two decades, pencil beam scanning (PBS) proton therapy experienced remarkable developments and has become a credible option in radiotherapy to treat certain types of cancers. However, anatomical variations occurring during irradiation, including tumor shrinkage and deformation, can be significant and can result in suboptimal patient treatment. The dosimetric uncertainty can be minimized through the use of motion mitigation techniques, which aim to mitigate the interplay effect between the motions in the patient and the beam delivery; the most common motion mitigation techniques are breath-hold, rescanning, and gating. However, these techniques are not efficient in terms of treatment delivery time. Hence it limits the patient throughput and increases the cost of the treatment.

The use of high-intensity proton beams to treat patients with ultra-high dose rates would open the possibilities of treating moving tumors. High-intensity beams could allow for efficient delivery of motion mitigation techniques. The use of ultra-high dose rates (UHDR) for all clinically used beam energies (70-230 MeV) could allow achieving FLASH proton therapy using Bragg peaks. In addition, hypo-fractionation and shorter treatment delivery times may increase patient throughput, lowering the cost of proton therapy treatment.

Therefore, in the last few years, the potential of UHDR for cancer treatment is being explored due to its potential for enhancing biological effect, faster hypo-fractionated treatments, and efficient motion mitigation.

The majority of proton therapy facilities produce energetic protons with cyclotrons. However, energy degradation for UHDR irradiations using cyclotrons is far from optimal. The issue is the need to lower the beam energy from the fixed value provided by the cyclotron (250/230 MeV) to the one needed for the treatment. Usually, this is obtained by letting the beam pass through a so-called energy selection system (ESS). First, the beam passes through a certain thickness of a low atomic number material (so-called 'degrader'), losing energy until the desired energy is reached. Unfortunately, this causes additional scattering and increases the energy spread of the beam. For this reason, a degrader system is always followed by a phase space selection system and energy analyzing dipole magnets, which selects the emittance of the beam and the right energy, lowering the momentum spread. Energy selection dipole magnets are dedicated to fine energy selection by filtering the desired momentum spread resulting from the energy degrader. Between the energy selection dipole magnets, it is required to use the two pairs of quadrupole magnets for the beam focusing purpose and the adjustable slit in the dispersive (bending) plane to select the desired momentum bend.

Such an energy modulation system has the drawback of introducing an energy-dependent beam current transmission due to the beam losses at the ESS. The design of ESS and these losses are described in Maradia et al., 2021. At PSI, for the lowest energies, the transmission through the energy selection system (which follows a graphite degrader) is less than 1%. In proton therapy, these low transmissions for lower energies cause an undesirable increase in treatment time, which complicates the treatment of moving tumors and it is not possible to achieve ultra-high dose rates to achieve FLASH proton therapy.

Alternatively, if energy degradation is performed downstream, just before the patient, it is possible to achieve high-intensity beams for low energy beams. However at the cost of the compromised beam width and size of the distal and lateral penumbra which is not optimal for patient treatment.

As mentioned above, cyclotron-based facilities need an energy degrader and an ESS. The momentum/energy spread after degrader for lower energies is very large which makes it impossible to transport a particle beam with this spread through the gantry beamline. To prevent losses along the beamline, it is unavoidable to use an energy selection dipole pair and momentum selection slit to limit the momentum spread such that it could be transported through the gantry beamline.

As an example, the ESS beamline of the PROScan facility at the Paul Scherrer Institute (PSI), located at CH-5232 Villigen PSI, Switzerland, is used. The system is illustrated in Fig. 1. At the exit of cyclotron 11, a quadrupole triplet 12 focuses the 250 MeV beam at the center of the degrader 13. After the degrader, a first collimator 14 selects the beam size, and the second collimator 15 selects the beam divergence. Then, the beam passes the following energy selection system ESS, consisting of two 58° bending magnets 18, several quadrupoles 19 and 21, and slits 20 for the momentum selection.

For lower energy beams the momentum spread after the degrader 13 is about ±5%. The bending magnet 18 was used to sweep the beam over the momentum slit aperture in the dispersive focus to select the desired momentum spread. Generally, in cyclotron-based proton therapy gantries, one chooses about 0.6% Δp/p by cutting the undesired momentum spread using the slits. This results in a factor 6-10 times decrease in the beam intensity depending on energy.

It is therefore the objective of the present invention to provide a particle beam transport system for the delivery of particle beam therapy to a patient that has a high transmission even at low beam energies in order to achieve appropriate treatment conditions, such as treatment time and dose rate.

This objective is achieved according to the present invention by a particle beam transport system for the delivery of particle beam therapy to a patient, comprising:
a) a particle beam source, such as an ion- or proton accelerator,
b) a beam transport section comprising a number of beam bending and/or beam focusing magnets for guiding the particle beam at a predefined energy and beam emittance to a patient therapy section, and
c) said patient therapy section comprising further beam bending and/or beam focusing magnets enabling the application of the particle beam to a predefined volume in the patient, wherein:
d) the beam transport section comprises an momentum reduction section disposed downsteam of a beam bending magnet; said momentum reduction section comprises an energy attenuation member being disposed within the particle beam in a way that different momentum particles of the particle beam see different thicknesses of material of the energy attenuation member thereby having different energy losses in the energy attenuation member resulting in a particle beam having a homogenous momentum distribution (p-Δp) over its cross-sectional area downstream of the energy attenuation member.

This measures particularly allow to achieve a high particle beam transmission already at low beam energies with a beam having an appropriate beam emittance for the therapy of the patient. By not cutting-off parts of the beam being apart from the desired beam spread, the present invention provides a way to present paths of different lengths to beam parts at the outer edges of the beam spread spectrum.

Suitably, the energy attenuation member may be disposed in a way that particles having a higher momentum have to pass a longer distance through the energy attenuation member as compared to particles having a lower momentum. Eventually, this measure results in a smooth energy distribution over the cross-sectional area of the particle beam thereby allowing a portion of the incoming beam to pass while having a favourable beam momentum spread in the desired range of surely lower than 1%.

In a preferred embodiment of the present invention, the energy attenuation member may have the form of a wedge.

Typically, the material of the energy attenuation member is selected from a low Z material, such as being selected from a group containing polyethylene, plexiglass, polymethyl methacrylate (PMMA), carbon, Boron carbide, and diamond.

In a further preferred embodiment of the present invention, the the patient therapy section may comprise a rotatable gantry, wherein the particle beam is transported by the beam transport section from the particle beam source to the rotatable gantry and wherein the beam transport section may comprise a degrader and an energy selection system including the enery attenuation member being disposed downstream of the degrader.

In order to enable the smoothing of the beam energy spread/momentum spread over the cross-sectional area of the particle beam for different beam energies, the energy attenuation member can be moved into the and out of the particle beam enabling to insert different thicknesses of the energy attenuation member based on the initial energy and the momentum spread of the particle beam.

In order to increase the versatility of the energy attenuation member, differently shaped energy attenuation members may be provided in predefined conditions to achieve a predefined momentum reduction. For example, it would be possible to provide different energy attenuation members for different ranges of the beam energy. Preferably, the differently shaped energy attenuation members each may have the form of a wedge, but of different dimensions and/or different materials.

Further preferred embodiment of the present invention are listed in the attached dependent claims.

Preferred embodiments of the present invention are hereinafter described in more detail with reference to the attached drawings which depict in:

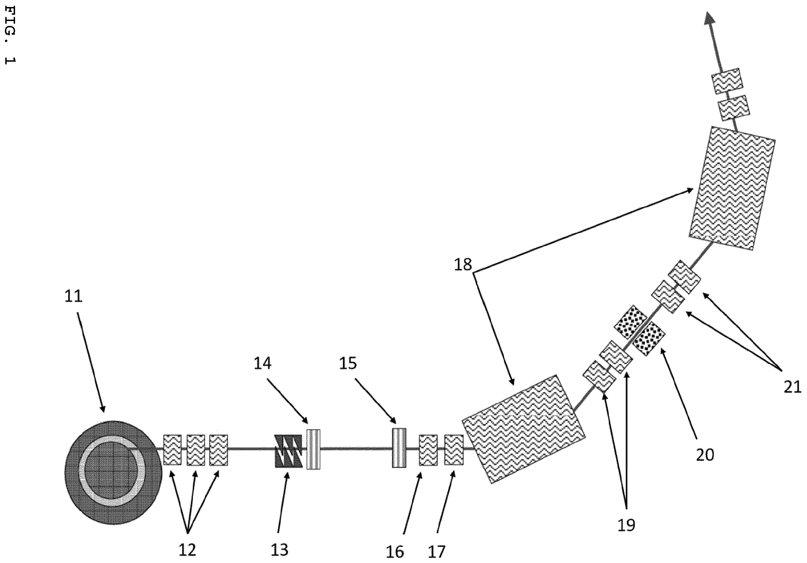

- Fig. 1: schematically a configuration of the energy selection system (ESS) of a medical particle beam facility according to the prior art;
- Fig. 2: schematically the principle of momentum selection using slits in the prior art
- Fig. 3: schematically the principle of momentum selection using a beam enery attenuation member in form of a wedge;
- Fig. 4: beam transmission from the cyclotron to isocenter by using the wedge according to Fig. 3, simulated at Paul Scherrer Institut, Energy selection system (ESS) of PROSCAN beam line, 5332 Villigen PSI, Switzerland; and
- Fig. 5: dose rate at the central axis of the beam with high transmission achieved using the wedge in the ESS.

In the prior art, momentum selection slits are used to select the momentum spread by cutting the beam. For this reason, a very small part of the total intensity for lower energy beams is transported along the beamline, causing low beam current transmissions. The present invention provides a way to solve this problem. It is proposed to reduce the momentum spread of the beam without cutting it by using a beam energy attenuarion member, preferably made of low Z material instead of slits. Due to the beam passing through the bending magnet, at the location of the former momentum selection slits, a distribution of the momentum from p+Δp to p-Δp is present. By replacing the slits with a particular beam attenuation member, preferably in form of a wedge, different momentum particles will pass through the different thicknesses of material and will achieve almost the same momentum (p-Δp) after the wedge. By using this method of momentum cooling, it is possible to transport a higher number of particles through the beamline for lower energies. Therefore, the transmission is increased by factors.

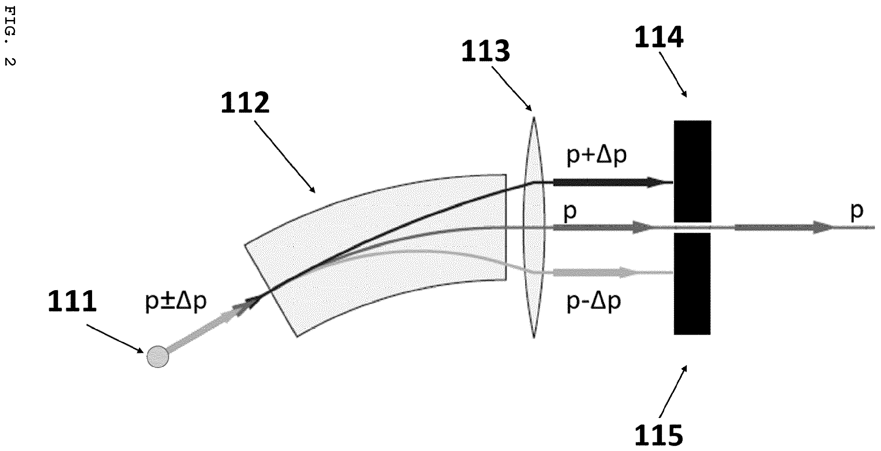

The present invention will now be described in detail in relation to the appended drawings. However, it is evident that a person skilled in the art may conceive several equivalent embodiments or other ways of executing the present invention. First, the principle of momentum selection using slits is disclosed, which is used in the prior art. Fig. 2 shows a layout of such an ESS according to the prior art. A source 111 of the particle beam has a momentum of p±Δp. Now, the beam will pass through the bending (dipole) magnet 112. Higher momentum particles (i.e. p+Δp) will bend less and lower momentum particles (i.e. p-Δp) will bend more while traveling through the bending magnet 112. In other words, the bend angle of a particle beam through a bending magnet is momentum dependent and particle beams with different momentum will disperse differently. This will lead to the broadening of the beam in the bending plane. After that a unit of focusing magnet 113 (two quadrupole magnets) is used to focus the beam at the momentum selection slits 114 and 115. By moving the slits 114 and 115 in the bending plane, one could choose different momentum bands. To achieve sharp distal penumbra for the patient treatment, it is important to choose the momentum spread of less than ±1 %. However, after the energy degrader, for lower energy beams, the momentum spread of the beam is in the range of ±5%. This introduces a significant beam loss in slits.

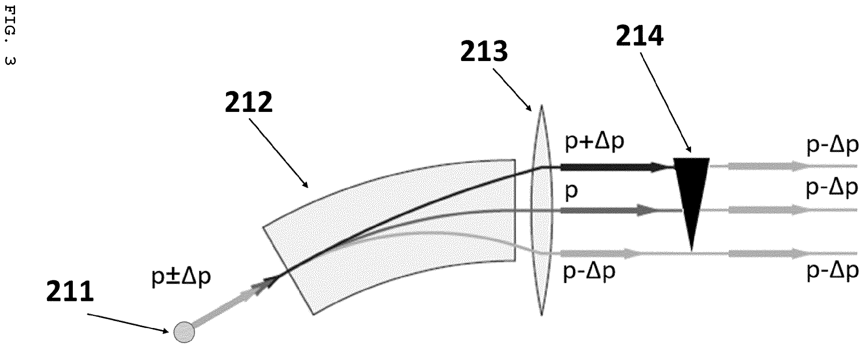

In the present invention, it is proposed to use a beam enery attenuation member, preferably in the form of a wedge 214, instead of slits in the ESS to achieve the desired momentum cooling without introducing a significant loss of particles. Fig 3 illustrates the working principle of the invention. In this invention, a bending magnet 212 is used to bend the particle beam and to achieve the distribution of the beam's momentum. After that to focus the beam at the location of the wedge, a focusing unit 213, i.e. two quadrupole magnets, is used. The thickness of the wedge 214 is chosen in a way that the different momentum particles will see different thicknesses of material and will lose different energy in the wedge 214 to get the same momentum p-Δp after the wedge 214. For example, particles with p+Δp momentum will pass through more material to achieve p-Δp momentum after the wedge 214 (loss of 2* Δp). Particles with p momentum (center of the beam) will pass through almost half of the material compared to p+Δp momentum particles. However, both momentum particles will achieve almost the same momentum p-Δp after the wedge 214. In this way, one could achieve almost the same momentum particles after the wedge 214 without introducing significant beam losses.

In the present example, the wedge 214 is made from polyethylene. It could be also made form plexiglass, polymethyl methacrylate (PMMA), carbon, Boron carbide, dimond and/or mixtures thereof. The wedge has the following dimensions: length of the wedge in the X-plane (bending plane) is 4 cm and in Y-plane is 2 cm while the thickness of the wedge is about 3.5 mm. Of course, the effective dimensions are dependend from the material and the indicident beam energy and energy spread. Thus, it could be also possible to choose wedges of different dimensions and/or different materials. The wedge may also have 2D- or 3D-curved intersections. The beam energy attenuation member could also have the form of trapezoid or a triangle or other suitable form to present different thicknesses of the beam energy attenuation member to particles having a different beam energy.

For low energy beams, one could achieve the maximum transmission between cyclotron to the isocenter of the patient therapy section, by transporting maximum accepted emittance in both transverse planes after the degrader along with momentum cooling with the wedge 214.

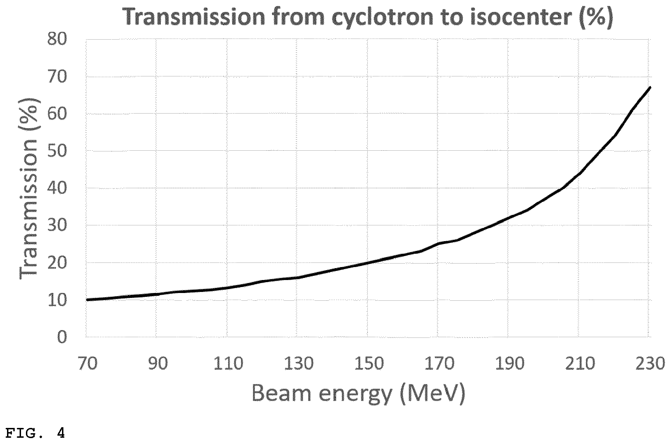

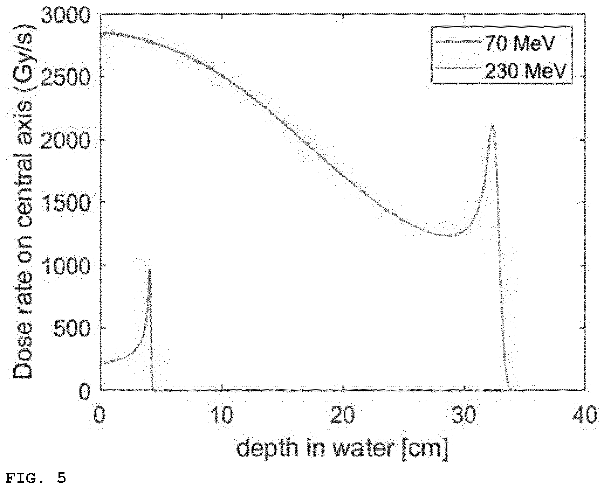

Fig 4 shows the achievable transmission simulated at Paul Scherrer Institut, Energy selection system (ESS) of PROSCAN beam line, 5332 Villigen PSI, Switzerland, by introducing the cooling of the beam momentum with the wedge 214. It is possible to achieve almost 10% transmission for 70 MeV beam while it is possible to achieve 67% transmission for 230 MeV beam. With these new transmissions (800 nA beam current from cyclotron), at Bragg peak (in water), it is possible to achieve a dose rate of ~950 Gy/s and ~2100 Gy/s for 70 MeV and 230 MeV beams respectively (Fig. 5).

In particle therapy facilities with a gantry, it is important to have the same emittance in both transverse planes at the entrance of the gantry to achieve gantry angle independent beam size at the isocenter. However, with the new ESS with the wedge 214, one gets different emittances in both transverse planes at the entrance of the gantry. To avoid this problem one could introduce the momentum cooling in terms of the beam energy attenuation member just after the energy degrader and use the emittance selection collimator to select the same emittance in both planes after the wedge 214. In this way, it is possible to achieve gantry angle independent beam size for patient treatment while achieving high-beam currents for low energy beams.

## Claims

1. A particle beam transport system for the delivery of particle beam therapy to a patient, comprising:
a) a particle beam source (11), such as an ion- or proton accelerator,
b) a beam transport section comprising a number of beam bending and/or beam focusing magnets for guiding the particle beam at a predefined energy and beam emittance to a patient therapy section, and
c) said patient therapy section comprising further beam bending and/or beam focusing magnets enabling the application of the particle beam to a predefined volume in the patient, wherein:
d) the beam transport section comprises an momentum reduction section disposed downsteam of a beam bending magnet; said momentum reduction section comprises an energy attenuation member being disposed within the particle beam in a way that different momentum particles of the particle beam see different thicknesses of material of the energy attenuation member thereby having different energy losses in the energy attenuation member resulting in a particle beam having a homogenous momentum distribution (p-Δp) over its cross-sectional area downstream of the energy attenuation member.

2. The particle beam transport system according to claim 1, wherein the energy attenuation member is disposed in a way that momentum particles having a higher momentum have to pass a longer distance through the energy attenuation member as compared to momentum particles having a lower momentum.

3. The particle beam transport system according to any of the preceding claims, wherein the energy attenuation member has the form of a wedge.

4. The particle beam transport system according to any of the preceding claims, wherein the material of the energy attenuation member is selected from a low Z material.

5. The particle beam transport system according to claim 4, wherein the low Z material is selected from a group containing polyethylene, plexiglass, polymethyl methacrylate (PMMA), carbon, Boron carbide, and diamond.

6. The particle beam transport system according to any of the preceding claims, wherein the patient therapy section comprises a rotatable gantry, wherein the particle beam is transported by the beam transport section from the particle beam source to the rotatable gantry and wherein the beam transport section comprises a degrader (13) and an energy selection system (ESS) including the enery attenuation member being disposed downstream of the degrader (13).

7. The particle beam transport system according to any of the preceding claims, wherein the energy attenuation member is movable into the and out of the particle beam enabling to insert different thicknesses of the energy attenuation member based on the initial energy and the momentum spread of the particle beam.

8. The particle beam transport system according to any of the preceding claims, wherein differently shaped energy attenuation members are provided in predefined conditions to achieve a predefined momentum reduction.

9. The particle beam transport system according to claim 8, wherein the differently shaped energy attenuation members each have the form of a wedge, but of different dimensions.
